# EUROPEAN PATENT APPLICATION

(11) **EP 2 308 518 A1**
(43) Date of publication of application: **13.04.2011**
(21) Application number: 09773156.6
(22) Date of filing: 29.06.2009
(51) Int. Cl.: A61L 27/00

(54) **BONE DEFECT FILLER NOT ADSORBING BONE GROWTH FACTOR AND NOT INHIBITING THE ACTIVITY OF THE SAME**

(30) Priority: 30.06.2008 JP 2008171896
(71) Applicant: The University of Tokyo, Bunkyo-Ku Tokyo 113-8654 (JP); Next 21 K.K., Tokyo 113-0033 (JP)
(72) Inventor: TEI, Yuichi, Tokyo 113-8654 (JP); SASAKI, Nobuo, Tokyo 113-8654 (JP); SUZUKI, Shigeki, Tokyo 113-0033 (JP)
(74) Representative: Ellis, Katherine Elizabeth Sleigh
(86) International application number: PCT/JP2009/002989
(87) International publication number: WO 2010/001578

(57) **Abstract**

An object of the invention is to provide a bone filling material capable of promoting the bone regeneration effect of a growth factor such as platelet rich plasma (PRP).

Inventors of the invention anticipated that the bone filling material adsorbs a growth factor like PRP, and the combined use of the bone filling material with PRP, the bone regeneration effect of PRP might be impaired. Further, the invention is basically related to the inhibition of this adsorbing effect of the bone filling material by using a capping agent. Since the adsorbing effect of the bone filling material is thus inhibited, PRP can exert the bone regeneration effect thereof in the case of using the bone filling material together with PRP.

## Description

### TECHNICAL FIELD

The present invention relates to a bone filling material, etc. which does not inhibit bone growth promoted by a bone growth factor. Explained more specifically, the invention relates to a bone filling material which does not inhibit the activity of a bone growth factor contained in body fluid effused after implant of a bone filling material or in platelet rich plasma.

### BACKGROUND ART

For the purpose of restoration of a bone defect part, etc., a bone filling material is used. For example, Japanese Patent Application Laid-Open (JP-A) No. 8-224261 discloses a bone filling material having a plurality of protruding parts.

Meanwhile, platelet rich plasma (hereinbelow, also referred to as "PRP") is known to contain a growth factor (or a proliferation factor) which promotes bone growth. Furthermore, a technology of using PRP together with a bone filling material has been recently suggested (for example, JP-A Nos. 2007-105186 and 2006-230683).

However, in reality, the bone regeneration effect of PRP is not necessarily always shown when a bone filling material is used in combination with PRP.

### PRIOR ART DOCUMENTS

### PATENT DOCUMENTS

Patent Document 1: JP-A No. 2007-105186
Patent Document 2: JP-A No. 2006-230683

### DISCLOSURE OF THE INVENTION

### PROBLEMS TO BE SOLVED BY THE INVENTION

Object of the invention is to provide a bone filling material which does not inhibit a bone regeneration effect of a growth factor contained in PRP, etc.

### MEANS FOR SOLVING THE PROBLEMS

The bone filling material promotes bone regeneration. Thus, conventionally it has never been thought that the bone filling material inhibits the activity of a growth factor, etc. However, inventors of the invention considered that the growth factor, etc. contained in PRP, for example, is adsorbed by a bone filling material, and as a result the bone regeneration effect of PRP is impaired even when PRP is used in combination with the bone filling material. The invention is basically based on the finding that, when a bone filling material is administered to a patient while a site of an artificial bone for the bone filling material to which a growth factor is adsorbed is blocked by a surface treatment agent, cell proliferation is promoted. Further, it is based on the finding that the cell proliferation can be effectively promoted by using the bone filling material in combination with PRP. The invention relates to the control of adsorption effect of the bone filling material by using a surface treatment agent. Thus, as the adsorption effect of the bone filling material is inhibited, PRP can exhibit the bone regeneration effect when the bone filling material is used in combination with PRP. Further, by using such the bone filling material, adsorption of PRP or a growth factor, etc. present in a living body to the bone filling material is inhibited, and therefore the bone regeneration can be promoted.

The first aspect of the invention is a bone filling material containing an artificial bone for the bone filling material containing a calcium-based material, a surface treatment agent coated on the artificial bone for the bone filling material, or a surface treatment agent which is impregnated in the artificial bone for the bone filling material. According to the bone filling material, the adsorption of a growth factor is inhibited by the surface treatment agent, and as a result, inhibition of the activity of the growth factor that is caused by adsorption of the growth factor on the artificial bone for the bone filling material at the time of administration of the bone filling material is prevented.

The preferred embodiment of the first aspect of the invention is a bone growth factor wherein the growth factor is contained in body fluid which infiltrates an administration site to which the bone filling material is administered. As shown in the examples described below, the bone filling material of the invention has an inhibited adsorptivity for the growth factor. Thus, as it is considered that the adsorption of a bone growth factor contained in body fluid is also effectively inhibited, the bone filling material of the invention may be suitably used as a bone filling material which does not impede bone regeneration.

The preferred embodiment of the first aspect of the invention is the bone filling material wherein the growth factor is a growth factor contained in PRP.

The preferred embodiment of the first aspect of the invention is the bone filling material wherein the artificial bone for the bone filling material containing a calcium-based material is a sintered body.

The preferred embodiment of the first aspect of the invention is the bone filling material wherein the weight ratio between the artificial bone for the bone filling material and the surface treatment agent is 1×10² : 1 to 1×10¹⁰ : 1. By having the ratio of a capping agent compared to the bone filling material within the range of 1×10² : 1 to 1×10¹⁰ : 1, the effect of the capping agent, i.e., to block an adsorption site for growth factor contained in the artificial bone for the bone filling material, can be effectively obtained. By having the ratio above, the bone filling material which effectively inhibits adsorption of the growth factor is provided. Further, with the bone filling material having the ratio above, adsorption of the bone growth factor contained in body fluid which infiltrates an administration site to which the bone filling material is administered can be effectively inhibited. In addition, as shown in the examples given below, bone regeneration can be effectively promoted by using the bone filling material of the invention.

The preferred embodiment of the first aspect of the invention is the bone filling material wherein the calcium-based material is one kind or a mixture of more than one kind selected from hydroxyl apatite, carbonic acid apatite, fluorapatite, chlorapatite, β-TCP, α-TCP, calcium metaphosphate, tetra-calcium phosphate, calcium hydrogen phosphate, calcium dihydrogen phosphate, calcium pyrophosphate, calcium carbonate, calcium sulfate, the salts thereof, or the solvates thereof. A porous artificial bone for the bone filling material may be obtained by using the calcium-based material. Accordingly, the capping agent can be infiltrated not only on the surface but also into the inside of an artificial bone for the bone filling material. As shown in the examples given below, by impregnating a capping agent into the artificial bone for the bone filling material containing a calcium-based material, a bone filling material which effectively promotes cell proliferation can be obtained. Further, as shown in the examples given below, the bone regeneration can be promoted by using the bone filling material of the invention.

The preferred embodiment of the invention is the bone filling material wherein the shape of the artificial bone for the bone filling material is any one of a granule type, a block type base, an order made base, an implant for dental root, or a sclerosing type artificial bone.

The preferred embodiment of the first aspect of the invention is the bone filling material described above wherein the artificial bone for the bone filling material is a granule type having a plurality of protruding parts with the diameter size of the sphere which can accommodate the artificial bone for the bone filling material is 1×10⁻² mm to 5 mm. By using the artificial bone for the bone filling material which has a shape having a plurality of protruding parts, the bone filling materials can be combined with each other to prevent their movement when they are administered to an affected site, and therefore they become a desirable cellular basis for bone regeneration. Further, as the surface area of the bone filling material to which cells can adsorb increases, the bone regeneration is promoted. As shown in the examples given below, by using the bone filling material having a shape with a plurality of protruding parts, cell proliferation can be effectively promoted. In addition, the bone regeneration can be promoted by using the bone filling material.

The preferred embodiment of the first aspect of the invention is the bone filling material wherein the surface treatment agent is one kind or a mixture of more than one kind of an amino acid, a peptide, a polysaccharide, a disaccharide, a chelating agent, a coupling agent, and a cross-linking agent.

The preferred embodiment of the first aspect of the invention is the bone filling material wherein the chelating agent is one kind or a mixture of more than one kind selected from a group consisting of a linear-chain polyphosphoric acid chelating agent, gluconic acid, asparaginic acid, ethylenediamine tetraacetic acid, metaphosphoric acid, citric acid, malic acid, nitrilo triacetic acid, and methyl glycine diacetic acid.

The preferred embodiment of the first aspect of the invention is the bone filling material wherein the capping agent is one or more kinds of an amino acid, a peptide, a polysaccharide, a disaccharide, lectin, proteoglycan, a glycoprotein, and a glycolipid.

The preferred embodiment of the first aspect of the invention is the bone filling material wherein the coupling agent is an aluminate-based coupling agent, a titanol-based coupling agent, or a silanol-based coupling agent.

The preferred embodiment of the first aspect of the invention is the bone filling material wherein the surface treatment agent is trehalose, serine, or dextran. As shown in the examples given below, by containing trehalose, serine, or dextran, cell proliferation is effectively improved. Thus, as being capable of promoting bone regeneration, the bone filling material of the invention may be used for filling a bone as appropriate.

The preferred embodiment of the first aspect of the invention is the bone filling material wherein the surface treatment agent is trehalose. As shown in the examples given below, by using the bone filling material which contains a solution of trehalose, the cell proliferation can be effectively promoted. Further, as shown in the examples given below, by using the bone filling material of the invention, the bone regeneration can be promoted.

According to the preferred embodiment of the first aspect of the invention, the bone filling material includes a bone growth agent coated on the artificial bone for the bone filling material, or a bone growth agent impregnated in the artificial bone for the bone filling material described above. As the bone filling material of the invention does not adsorb a growth factor, the bone regeneration can be effectively promoted with incorporation of the bone growth agent including a growth factor. As shown in the examples given below, by using the bone filling material and the bone growth agent including a growth factor, the cell proliferation can be effectively promoted. By using these bone growth agent and the bone filling material, the bone regeneration can be promoted.

According to the preferred embodiment of the first aspect of the invention, the bone filling material includes PRP coated on the artificial bone for the bone filling material or PRP impregnated in the artificial bone for the bone filling material. As the adsorption of the growth factor is under control, the bone filling material of the invention does not inhibit the activity of the growth factor in PRP. Accordingly, by using the bone filling material containing PRP, the bone regeneration can be effectively promoted.

The second aspect of the invention relates to a method of producing a bone filling material including infiltrating a surface treatment agent into an artificial bone for a bone filling material and incorporating a bone growth agent in the artificial bone for the bone filling material to which the surface treatment agent has been infiltrated. The step of infiltrating a surface treatment agent into an artificial bone for a bone filling material is to coat the artificial bone for the bone filling material with a surface treatment agent or to impregnate the artificial bone for the bone filling material in a solution of a surface treatment agent. The step of incorporating a bone growth agent is to coat the artificial bone for the bone filling material to which the surface treatment agent has been coated or infiltrated with a bone growth agent, or to impregnate the artificial bone for the bone filling material to which the surface treatment agent has been coated or infiltrated in a solution of a bone growth agent. The artificial bone for the bone filling material contains a calcium-based material. According to the method above, the bone filling material capable of promoting a bone regeneration effect can be produced.

The third aspect of the invention is a medical kit including a vessel for preparing PRP. According to the invention, the bone filling material, the surface treatment agent, PRP, and a vessel for preparing PRP are included in the kit. The adsorptivity of the growth factor in PRP is controlled by the surface treatment agent, and as a result, inhibition of the activity of the growth factor that is caused by adsorption of the growth factor on the artificial bone for the bone filling material at the time of administration of the bone filling material is prevented. For using the kit for preparing PRP, the surface treatment agent and PRP are accommodated in the vessel for PRP and impregnated into the bone filling material, or the surface treatment agent and PRP accommodated in the vessel for PRP are coated on the bone filling material, and thus PRP is incorporated in the bone filling material.

### EFFECTS OF THE INVENTION

According to the invention, the bone filling material which does not inhibit the bone regeneration effect of a growth factor is provided.

### BRIEF DESCRIPTION OF THE DRAWINGS

Fig. 1 is a conceptual diagram for explaining an example of a tetrapod-shaped bone filling material.
Fig. 2 is a graph, in place of a diagram, illustrating the effect of a tetrapod type artificial bone on cell proliferation, wherein the artificial bone has been immersed in a solution of each capping agent including trehalose, L-serine, and dextran 40.
Fig. 3 is a graph, in place of a diagram, illustrating the effect of the trehalose treatment on the adsorptivity of the bone filling material for a growth factor.
Fig. 4 is a graph, in place of a diagram, illustrating the effect of PRP on proliferation of MC3T3-E1 cells, which are osteoblast-like cells.
Fig. 5 is a graph, in place of a diagram, illustrating the effect of a tetrapod type bone filling material on the cell proliferative activity of PRP, wherein the bone filling material has been impregnated in an aqueous solution of trehalose.

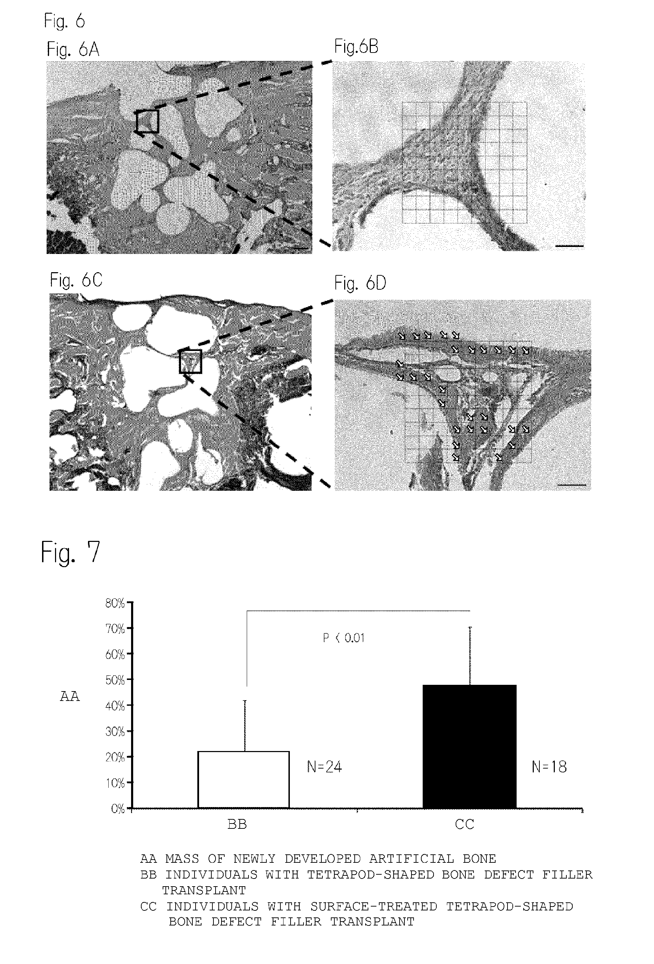

Fig. 6 includes photographic images, in place of a diagram, illustrating the effect of surface treatment of the bone filling material on the bone regeneration in a living body. Fig. 6A is a photographic image, in place of a diagram, illustrating the tissue section of a rat femur bone in which the tetrapod type bone filling material with no surface treatment has been implanted. Fig. 6B is a photographic image, in place of a diagram, wherein a grid consisting of one hundred blocks is formed on the enlarged image of the tissue section shown in Fig. 6A. Fig. 6C is a photographic image, in place of a diagram, illustrating the tissue section of a rat femur bone in which the bone filling material with the surface treatment has been implanted. Fig. 6D is a photographic image, in place of a diagram, wherein a grid consisting of one hundred blocks is formed on the enlarged image of the tissue section shown in Fig. 6C.
Fig. 7 is a graph that shows the bone regeneration.

### BEST MODE FOR CARRYING OUT THE INVENTION

The first aspect of the invention is a bone filling material including an artificial bone for the bone filling material containing a calcium-based material, a surface treatment agent coated on the artificial bone for the bone filling material, or the surface treatment agent which is impregnated in the artificial bone for the bone filling material. According to the bone filling material, the adsorptivity of a growth factor is inhibited by the surface treatment agent, and as a result, inhibition of the activity of the growth factor that is caused by adsorption of the growth factor on the artificial bone for the bone filling material at the time of administration of the bone filling material is prevented. Hereinbelow, each element of the invention is explained.

### [Bone Filling Material]

The bone filling material is to be administered or is to be embedded as an implant to a bone defect site or a bone deformation site. It has a characteristic of being replaced with bone tissues gradually. The adsorptivity between a bone growth agent and a bone filling material can be controlled by inactivating a functional group of bone filling material by a surface treatment agent, and therefore, adsorption of the growth factor included in the bone growth agent on the bone filling material can be prevented.

### [Artificial Bone for the Bone Filling Material]

The artificial bone for the bone filling material is prepared with a composition containing a calcium-based material, and it is a body having a granule shape or a block shape, for example. The shape of the artificial bone for the bone filling material is not limited to the shapes described above, and examples thereof include an order made base, an implant for dental root, or a sclerosing type artificial bone, in addition to a granule type and a block type base. Examples of the granule type artificial bone for the bone filling material include a tetrapod type in which a plurality of protrusions is included. In such case, a plurality of the bone filling materials is administered to an affected site. Further, the bone filling material may have a shape which is designed considering a patient's bone shape like an implant (i.e., order made substrate). Generally, such bone filling material is embedded in an affected site by a surgical operation, etc., and the material is gradually replaced with bone tissues.

### [Method of Producing Artificial Bone for the Bone Filling Material]

An artificial bone for the bone filling material is well known. Hereinbelow, an example of method of producing an artificial bone for the bone filling material is briefly explained. The method of producing an artificial bone for the bone filling material includes a kneading step, a molding step, a binder removing (degreasing) step, and a sintering step. The kneading step is a step to knead a raw material including a calcium-based material and a material including a binder. The molding step is a step to obtain a molded body having a pre-determined shape from the kneaded product resulting from the above kneading step by extrusion molding using an extrusion molding machine with a mold. The binder removing (degreasing) step is a step to remove the binder included in the molded body obtained from the molding step, and therefore to obtain a degreased body. The sintering step is a step to obtain a sintered body by heating and sintering the degreased body after the binder removing step. Further, steps like a post treatment step to perform a post treatment of the molded body may be included as appropriate.

According to the preferred embodiment of the first aspect of the invention, the artificial bone for the bone filling material containing the calcium-based material is a sintered body. The sintered body may be obtained by performing the sintering step for producing the artificial bone for the bone filling material as described above. The sintering temperature for obtaining the sintered body of the invention may be 500 to 2000°C. The sintering time may be 1 hour to 60 hours. The sintering time is appropriately adjusted depending on the sintering temperature and the artificial bone for the bone filling material containing the calcium-based material. By using the sintered body, hardness of the bone filling material increases, and therefore it can be used more appropriately as a bone filling material.

The preferred artificial bone for the bone filling material of the invention has a shape with a plurality of protruding parts. And it is more preferred that the protruding parts be provided so that they are arranged in linear symmetry, plane symmetry, or spatial symmetry. A specifically preferred shape of the artificial bone for the bone filling material is a tetrapod-shape (a shape having four protruding parts extending toward each vertex from the center of the regular tetrahedron), or a shape having n (n = 6, 8, and 12, etc.) protruding parts extending toward each vertex from the center of the regular body having n faces. The size of the bone filling material (the diameter of a sphere that the bone filling material can be accommodated) is, for example, from 1 × 10⁻² mm to 5 mm, preferably from 5 × 10⁻² mm to 3 mm, more preferably from 1 × 10⁻¹ mm to 2 mm, and still more preferably from 2 × 10⁻¹ mm to 1.5 mm. The shape of the bone filling material reflects the shape of the artificial bone for the bone filling material. Hereinbelow, preferred shapes of the bone filling material of the invention are explained in view of the drawing.

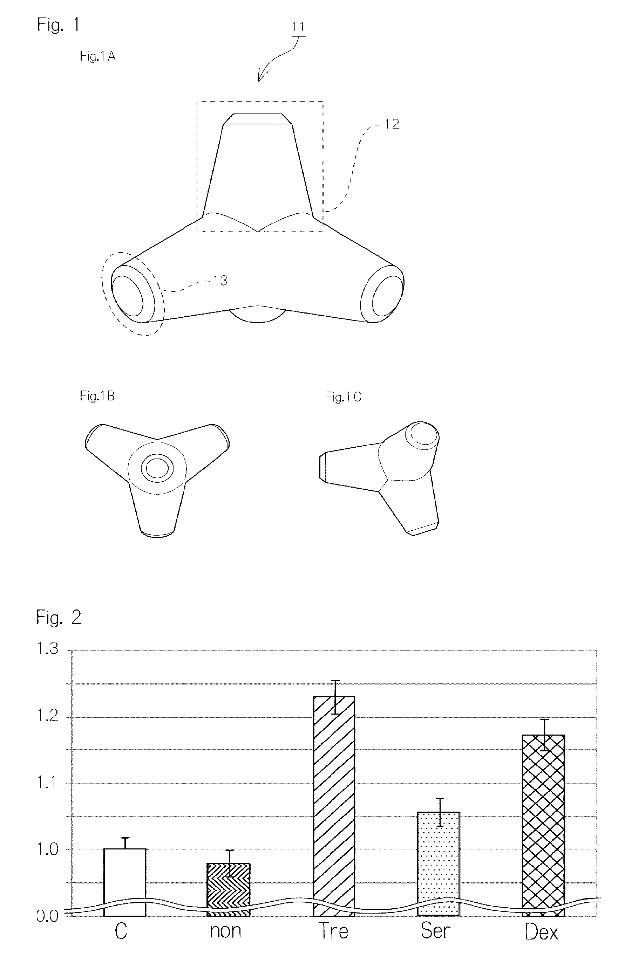

Fig. 1 is a conceptual diagram for explaining an example of a tetrapod-shaped artificial bone for the bone filling material. Fig. 1A is a side view, Fig. 1B is a top view, and Fig. 1C is a perspective view. Note that "Fig" in the drawings means "Figure" (the same below). The bone filling material (11) shown in Fig. 1A to Fig. 1C is a tetrapod type (a shape having four protruding parts (12) extending toward each vertex from the center of the regular tetrahedron) artificial bone for the bone filling material. Further, it is preferred that a taper is provided at the tip part (13) of each protruding part (12) and is smoothly shaped (the same below). As shown in Fig. 1A to Fig. 1C, each protruding part (12) has substantially the same shape, but one or two of them may be small shaped. Also, each protruding part has, for example, a truncated cone shape which is tapered toward the tip end (13) thereof. The tip portion of each protruding part may be hemispheric.

### [Calcium-Based Material]

In general, the calcium-based material is the main component of the bone filling material. The calcium-based material is not specifically limited if it is close to bone components. Examples of the calcium-based material include calcium phosphate-based material, calcium carbonate-based material, calcium lactate, and calcium gluconate. Among them, calcium phosphate-based material or calcium carbonate-based material is preferred. Specific examples of the calcium phosphate-based material as powdered ingredients include one or more kinds of hydroxyl apatite, carbonic acid apatite, fluorapatite, chlorapatite, β-TCP, α-TCP, calcium metaphosphate, tetra-calcium phosphate, octa-calcium phosphate, calcium hydrogen phosphate, calcium dihydrogen phosphate, calcium pyrophosphate, the salts thereof, or the solvates thereof. Among them, β-TCP or hydroxyl apatite is preferred. Specific examples of the calcium carbonate-based materials include calcium carbonate and calcium hydrogen carbonate. Among them, calcium carbonate is preferred. Chemical compound other than the above may be included in the calcium-based material as needed if the above chemical compound is the main component of the calcium-based material. By using the calcium-based material, a porous bone filling material can be produced. As such efficiently including a capping agent and a bone growth agent, the porous bone filling material may effectively promote the bone regeneration.

### [Surface Treatment Agent]

In order to block the adsorption site on the artificial bone for the bone filling material, the surface treatment agent is infiltrated by coating or impregnation to the artificial bone for the bone filling material. Examples of the surface treatment agent include one kind or a mixture of more than one kind of a chelating agent, a capping agent, a coupling agent, and a cross-linking agent. The surface treatment agent of the invention may be dissolved in a well known solution which can dissolve the surface treatment agent. Preferably, however, it is water, physiological saline, or alcohol, etc. Concentration of the surface treatment agent is not specifically limited if it is a concentration at which the surface treatment agent can be dissolved. However, if the concentration is too high, viscosity increases and infiltration of the surface treatment agent to the artificial bone for the bone filling material is difficult to obtain under normal pressure, thus it is desirable to perform under reduced or increased pressure. If the infiltration of the surface treatment agent is performed under reduced or increased pressure, it becomes possible to achieve the infiltration of the solution of the surface treatment agent with high viscosity (high concentration) in shorter time compared to the infiltration of the surface treatment agent under normal pressure. Conditions for the infiltration of the artificial bone for the bone filling material with a solution of the surface treatment agent by immersion under reduced or increased pressure may be appropriately determined by a skilled person in the pertinent art depending on properties of the solution of the surface treatment agent or the artificial bone for the bone filling material. The surface treatment agent of the invention may be used as a mixture of two or more different surface treatment agents. Mixing ratio of the surface treatment agent is not specifically limited. It may be mixed with the same ratio or a skilled person in the pertinent art can adjust the mixing ratio depending on properties of the surface treatment agent used. By mixing the surface treatment agents, adsorption sites which can be blocked increase, and therefore the bone filling material having higher surface treatment effect than a case in which only a single agent is used can be obtained.

### [Chelating Agent]

Examples of the chelating agent in the invention include one kind or a mixture of more than one kind selected from linear polyphosphoric acid chelating agent, gluconic acid, asparaginic acid, ethylenediamine tetraacetic acid (EDTA), metaphosphoric acid, citric acid, malic acid, nitriloacetic acid, methylglycine diacetic acid, 1,2-cyclohexanediamine tetraacetic acid, diethylenetriamine acetic acid, 2-hydroxyethylethylenediamine triacetic acid, triethylene tetramine hexaacetic acid, dimethylglyoxime, dithizone, oxine, acetylacetone, or their pharmaceutically acceptable salts. Among them, one kind or a mixture of more than one kind selected from linear polyphosphoric acid chelating agent, gluconic acid, asparaginic acid, ethylenediamine tetraacetic acid (EDTA), metaphosphoric acid, citric acid, malic acid, nitrilotriacetic acid, and methylglycine diacetic acid is preferable. Further, it is preferable to include gluconic acid. When the artificial bone for the bone filling agent is infiltrated with a chelating agent, concentration of the solution of chelating agent may be 0.1 to 40% by weight, 1 to 10% by weight, or 5 to 15% by weight under normal pressure. When the infiltration is performed under reduced or increased pressure, the concentration of more than 40% by weight may be also employed if it is a concentration at which the chelating agent can be dissolved. Conditions for the reduced or increased pressure may be appropriately determined depending on the material of the bone filling agent, and viscosity of the solution of a chelating agent, etc.

### [Capping Agent]

As a capping agent of the invention, it is preferable to include at least one kind of an amino acid, a peptide, a polysaccharide, a disaccharide, lectin, proteoglycan, a glycoprotein, and a glycolipid.

Examples of the amino acid included in the capping agent include one of an amino acid selected from the group consisting of "alanine, arginine, asparagine, asparaginic acid, cysteine, glutamine, glutamic acid, glycine, histidine, isoleucine, leucine, lysine, methionine, phenylalanine, proline, serine, threonine, tryptophan, tyrosine, and valine", a derivative of amino acid, and a pharmaceutically acceptable salt thereof, or two or more kinds thereof. The amino acid is preferably a naturally occurring L form. Among the amino acids above, serine, threonine, tyrosine, cysteine, or methionine having good bonding property to the adsorption site in the artificial bone for the bone filling material is preferable. Serine is more preferable. When the infiltration is performed under normal pressure, concentration of the amino acid solution is preferably 0.01 to 10 mol/L, more preferably 0.05 to 5 mol/L, and still more preferably 0.1 to 2 mol/L. When the infiltration is performed under reduced or increased pressure, the concentration of more than 10 mol/L may be also employed if it is a concentration at which the amino acid can be dissolved. Conditions for the reduced or increased pressure may be appropriately determined depending on the material of the bone filling agent, and viscosity of the capping agent solution, etc.

Examples of the peptide included in the capping agent include a dipeptide, a tripeptide, a tetrapeptide, and a pentapeptide, that are formed of any combination with those selected from a group consisting of "alanine, arginine, asparagine, asparaginic acid, cysteine, glutamine, glutamic acid, glycine, histidine, isoleucine, leucine, lysine, methionine, phenylalanine, proline, serine, threonine, tryptophan, tyrosine, and valine", or one of their pharmaceutically acceptable salts or two or more types thereof. Among the peptides, the dipeptide or tripeptide having short peptide chain is preferable to obtain quick infiltration of the bone filling material with the capping agent. When the infiltration is performed under normal pressure, concentration of the peptide solution is preferably 0.01 to 10 mol/L, more preferably 0.05 to 5 mol/L, and still more preferably 0.1 to 2 mol/L. When the infiltration is performed under reduced or increased pressure, the concentration of more than 10 mol/L may be also employed if it is a concentration at which the peptide can be dissolved. Conditions for the reduced or increased pressure may be appropriately determined by a skilled person in the pertinent art depending on the material of the bone filling agent, and viscosity of the capping agent solution, etc.

Examples of the polysaccharide included in the capping agent include one or more kinds of pullulan, guar gum, lambda carrageenan, tragacanth gum, pectin, mannan, dextran, maltodextran, glucomannan, amylose, amylopectin, agarose, tamarind seed gum, carrageenan, gellan gum, carboxylmethyl cellulose, xanthan gum, carraya gum, gum Arabic, gati gum, arabinogalactan, curdlan, or their acid salts (for example, sulfate salt). Among them, preferred are pullulan, dextran, and maltose, and more preferred is dextran. When the infiltration is performed under normal pressure, concentration of the polysaccharide solution is preferably 1 to 40% by weight, more preferably 5 to 30% by weight, and still more preferably 15 to 25% by weight. When the infiltration is performed under reduced or increased pressure, the concentration of more than 40% by weight may be also employed if it is a concentration at which the polysaccharide can be dissolved. Conditions for the reduced or increased pressure may be appropriately determined by a skilled person in the pertinent art depending on the material of the bone filling agent, and viscosity of the capping agent solution, etc.

Examples of the disaccharide included in the capping agent include malt sugar (i.e., maltose), isomaltose, cellobiose, gentiobiose, nigerose, laminaribiose, cosibiose, sphorose, melibiose, lactose, turanose, sophorose, trehalose, isotrehalose, sucrose, saccharose, lactose, and isosaccharose. Among them, preferred are trehalose, isotrehalose, maltose, isomaltose, cellobiose, or gentiobiose, and more preferred is trehalose. When the infiltration is performed under normal pressure, concentration of the disaccharide solution is preferably 1 to 40% by weight, more preferably 5 to 30% by weight, and still more preferably 15 to 25% by weight. When the infiltration is performed under reduced or increased pressure, the concentration of more than 40% by weight may be also employed if it is a concentration at which the disaccharide can be dissolved. Conditions for the reduced or increased pressure may be appropriately determined by a skilled person in the pertinent art depending on the material of the bone filling agent, and viscosity of the capping agent solution, etc.

Examples of the glycoprotein included in the capping agent include proteoglycan, mucin, and avidin. Among them, preferred is proteoglycan. Examples of the proteoglycan include a complex between muco-polysaccharide and protein. Further, examples of the muco-polysaccharide include hyaluronic acid, chondroitin sulfate, heparan sulfate, keratan sulfate, dermatan sulfate, and heparin. Concentration of the glycoprotein solution is 1 to 30% by weight, and preferably 15 to 25% by weight. Examples of the glycolipid included in the capping agent include a galacto lipid, a sulfo lipid, a sphingo lipid (e.g., cerebroside and ganglioside), and glycosphingo phospholipid. Concentration of the glycolipid is 1 to 30% by weight, and preferably 10 to 20% by weight. The surface treatment can be performed effectively by using a solution having such concentration.

Examples of the coupling agent included in the surface treatment agent include an aluminate-based coupling agent, a titanol-based coupling agent, and a silanol-based coupling agent. Among them, preferred is a silanol-based coupling agent. Concentration of the coupling agent may be 1 to 15% by weight, and preferably 5 to 10% by weight. Further, according to the invention, it is preferable to dissolve the coupling agent in a weakly acidic solution (pH 4.5 to 6.5). By using a solution with such pH, the effect of the surface treatment agent may be improved more.

Examples of the cross-linking agent included in the surface treatment agent include glutaraldehyde, epoxide (e.g., bis-oxirane), oxidized dextran, p-azidobenzoyl hydrazine, N-[α-maleimidacetoxy]succinimide ester, p-azidophenylglyoxal-hydrate, bis-[β-(4-azidosalicylamide)ethyl]disulfide (BASED), bis[sulfosuccinimidyl]suberate, dithiobis[succinimidyl]propionate, disuccinimidyl suberate, 1-ethyl-3-[3-dimethylaminopropyl]carbodiimide hydrochloride, and ethoxylate (20) trimethylpropane triacrylate.
Concentration of the solution of the cross-linking agent may be 0.01 to 20% by weight, and preferably 0.1 to 10% by weight for performing the infiltration. Conditions for the reduced or increased pressure may be appropriately determined by a skilled person in the pertinent art depending on the material of the bone filling agent, and viscosity of the capping agent solution, etc.

### [Growth Factor]

Growth factor indicates a factor which functions as a regulator for cell proliferation or differentiation from early development, sustaining a life, to aging of an individual multicelullar animal. Specific examples include epidermal growth factor (EGF), insulin-like growth factor (IGF), transforming growth factor (TGF), vascular endothelial growth factor (VEGF), hepatocyte growth factor (HGF), platelet-derived growth factor (PDGF), embryonic smooth muscle myosin heavy chain (SMemb), bone morphogenetic protein (BMP), granulocyte colony-stimulating factor (G-CSF), erythropoietin (EPO), thrombopoietin (TPO), and basic fibroblast growth factor (b-FGF). Further, among the growth factors described above, the bone growth factor is a factor which is related to bone growth and may be produced in a living body.

According to the preferred embodiment of the first aspect of the invention, the growth factor is a bone growth factor which is included in body fluid infiltrating an administration site to which the bone filling material has been administered. Examples of the bone growth factor include EGF, TGF-β, IGF, VEGF, HGF, PDGF, SMemb, and BMP. These bone growth factors may be obtained by a well known method, and one kind or a mixture of two or more kinds may be used as a bone growth agent. As shown in the examples given below, because the adsorption site for growth factor on the artificial bone for the bone filling material is blocked by the surface treatment of the bone filling material, inhibition of the growth factor which is caused by the adsorption of the growth factor on the bone filling material is prevented, and therefor cell proliferation can be promoted.

According to the preferred embodiment of the first aspect of the invention, the solution containing the growth factor is PRP. As having a bone growth factor like EGF, TGF-β, and IGF, PRP is suitable as a bone growth agent. A solution containing PRP may be produced according to a well known method. For example, JP-A No. 2006-104106 can be mentioned. As shown in the examples given below, the bone filling material infiltrated with the surface treatment agent of the invention has inhibited adsorption of a growth factor. Further, as shown in the examples given below, by having the bone growth agent incorporated in the bone filling material of the invention, cell proliferation can be promoted. Further, as shown in the examples given below, by using the bone filling material containing the bone growth agent of the invention, bone regeneration effect can be promoted.

According to the preferred embodiment of the first aspect of the invention, the weight ratio between the artificial bone for the bone filling material and the surface treatment agent is 1×10² : 1 to 1×10¹⁰ : 1. If the ratio of the surface treatment agent is too small, reduction in the activity of the growth factor cannot be prevented. On the other hand, if the ratio of the surface treatment agent is too big, size of the bone filling material increases. As such, a sufficient amount of the bone filling material cannot be administered to an affected site, and thus sufficient strength cannot be obtained after the administration. Further, as the absorption of the bone filling material occurs slowly, replacement into a patient's own bone is slowed down, which may be a burden for the patient. Thus, according to the invention, the weight ratio between the artificial bone for the bone filling material and the surface treatment agent is preferably from 1×10² : 1 to 1×10¹⁰ : 1, more preferably from 1.5×10² : 1 to 1×10⁵ : 1, and still more preferably from 2×10² : 1 to 1×10³ : 1.

According to the preferred embodiment of the first aspect of the invention, the bone filling material further contains a bone growth agent coated on the artificial bone for the bone filling material or a bone growth agent infiltrated in the artificial bone for the bone filling material. The artificial bone for the bone filling material which is either coated or infiltrated with the bone growth agent may be obtained by the infiltration or coating with the bone growth factor as described below. As for the bone growth agent, it is preferable to use a mixture which contains one or more types of bone growth factor including EGF, TGF-β, IGF, VEGF, HGF, PDGF, SMemb, and BMP, or PRP, as described above. More preferably, it is PRP. As described above, PRP may be obtained by a well known method. It is considered that, by using PRP of a patient to whom administration is carried out, the bone growth progresses effectively after administration of the bone filling material.

When the bone filling material is administered to an affected site, body fluid infiltrates at the administration site. In such body fluid, a bone growth factor which is included in the bone growth agent is present. Since it takes time for the bone growth factor to show its activity after infiltration of body fluid, the bone growth may be slowed down if the amount of the bone growth agent contained in the bone filling material is small. On the other hand, if the amount of the bone growth agent is too high, desirable proliferation of osteoblast cells that are involved with bone growth is not expected. Thus, according to the invention, the weight ratio between the artificial bone for the bone filling material and the bone growth agent is preferably from 1×10² : 1 to 1×10⁵ : 1, more preferably from 5×10² : 1 to 5×10⁴ : 1, and still more preferably from 1×10³ : 1 to 1×10⁴ : 1. By having this weight ratio, each of the bone growth agent contained in the bone filling material and the bone growth factor contained in the body fluid which infiltrates an administration site may appropriately work to promote the bone growth.

The second aspect of the invention relates to a method of producing a bone filling material including infiltrating a surface treatment agent into an artificial bone for the bone filling material and incorporating a bone growth agent in an artificial bone for the bone filling material to which a surface treatment agent has been infiltrated. The artificial bone for the bone filling material can be produced according to the production method described above. According to the invention, the artificial bone for the bone filling material preferably contains the calcium-based material described above.

### [Method of Producing Artificial Bone for the Bone Filling Material]

Artificial bone for the bone filling material is well known. Hereinbelow, an example of producing an artificial bone for the bone filling material is briefly explained. The method of producing an artificial bone for the bone filling material includes a kneading step, a molding step, a binder removing (degreasing) step, and a sintering step. The kneading step is a step to knead a raw material including a calcium-based material and a material including a binder. The molding step is a step to obtain a molded body having a pre-determined shape from the kneaded product resulting from the above kneading step by extrusion molding using an extrusion molding machine with a mold. The binder removing (degreasing) step is a step to remove the binder included in the molded body obtained from the molding step, and therefore to obtain a degreased body. The sintering step is a step to obtain a sintered body by heating and sintering the degreased body after the degreasing step. Further, steps like a post treatment step to perform a post treatment of the molded body may be included as appropriate.

### [Step of Infiltrating with the Surface Treatment Agent]

The step of infiltrating the artificial bone for the bone filling material with a surface treatment agent is a step of coating the artificial bone for the bone filling material with a surface treatment agent, or a step of impregnating the artificial bone for the bone filling material in a solution of a surface treatment agent. With respect to the step of impregnating or coating with a surface treatment agent, it is not specifically limited if it is a method which enables the impregnation or coating of the artificial bone for the bone filling material with a surface treatment agent. For the step of producing the artificial bone for the bone filling material, the surface treatment agent may be mixed as a raw material. Specifically, the artificial bone for the bone filling material may be immersed in a solution of a surface treatment agent, and then by keeping it at room temperature and under normal pressure for 1 hour to 6 hours the surface treatment agent may become possible to get impregnated. Further, the step of impregnating a surface treatment agent may be performed under reduced or increased pressure. Conditions for the reduced or increased pressure may be appropriately determined by a skilled person in the pertinent art depending on the artificial bone for the bone filling material and a solution of a surface treatment agent that are used. It is also possible to perform a pre-drying or a sterilization treatment after the infiltration with a surface treatment agent. After that, the artificial bone for the bone filling material to which a surface treatment agent is either impregnated or coated may be dried.

### [Step of Impregnating or Coating the Bone Growth Agent]

The above-described step of incorporating a bone growth agent is a step of coating the artificial bone for a bone filling material which has been either coated or impregnated with a surface treatment agent with a bone growth agent, or a step of impregnating the artificial bone for the bone filling material which has been either coated or impregnated with a surface treatment agent in a solution of a bone growth agent. By appropriately impregnating or coating the artificial bone for the bone filling material which has been either coated or impregnated with the surface treatment agent with a bone growth agent, the bone filling material of the invention can be produced. The method for coating a bone growth agent includes immersion coating, spray coating, and spin coating, wherein a composition is obtained by dissolving the bone growth agent with well-known pharmaceutically acceptable diluent (solvent) and the resulting composition is coated. Among them, immersion coating is preferred. Immersion coating of the bone growth agent allows the impregnation of the bone growth agent to the surface or inside of the bone filling material. Namely, the invention can also provide a bone filling material to which a pre-determined bone growth agent is impregnated or coated. The bone growth agent may be coated to a pre-determined site by the ink jet method. In particular, the bone growth agent is coated to a certain designed site by an ink jet device. Further, when the bone filling material is produced by computer-supported design such as rapid prototype, the bone growth agent may be coated to the material according to a certain program or input information in the process of producing the bone filling material or the artificial bone for a bone filling base material. As for a bone growth agent, PRP is preferably used. As PRP includes bone growth factors like FGf, TGF-β, and IGF, it is preferred as a bone growth agent. Further, the bone growth agent of the kit of the invention may further include a growth factor. Examples of the growth factor include the same growth factors as described above. Thus, by incorporating the bone growth agent including a growth factor, bone regeneration can be effectively promoted. A skilled person in the pertinent art is able to adjust the necessary amount according to a known method by calculating an amount of PRP required for the impregnation or coating step.

### [Step of Drying the Surface Treatment Agent]

The preferred embodiment of the second aspect of the invention includes a step of drying the artificial bone for the bone filling material which has been infiltrated with the surface treatment agent. By performing the step of incorporating the bone growth agent after drying the artificial bone for the bone filling material which has been impregnated with the surface treatment agent, the bone growth agent can be homogeneously incorporated in the artificial bone for the bone filling material. The drying step can be appropriately adjusted depending on property of the surface treatment agent, etc., and keeping in a dryer at 30 to 100°C may be mentioned, for example. The drying time may be 2 min to 60 min, but it can be longer than 60 min. Keeping under air-blowing can be also carried out to shorten the drying time. The air-blowing speed can be 0.1 to 5 m/sec. However, if the air-blowing speed is too fast, unevenness may be caused. On the other hand, if the air-blowing speed is too slow, it takes too much time for drying, thus lowering the production efficiency. As such, it is preferably 0.2 to 3 m/sec, and preferably 0.5 to 1.5 m/sec.

### [Method of Using the Bone Filling Material]

The bone filling material produced by the production method of the invention is injected in bone defect sites, osteoporosis sites, or bone extension sites. Also, in addition to be filled in the gaps of bones such as bone defect sites, it can be also used as a carrier of a certain bone growth agent.

The third aspect of the invention is a medical kit including a vessel for preparing PRP. According to the invention, the bone filling material, the surface treatment agent, PRP, and a vessel for preparing PRP are included in the kit. The adsorptivity of the growth factor in PRP is controlled by the surface treatment agent, and as a result, inhibition of the activity of the growth factor that is caused by adsorption of the growth factor on the artificial bone for the bone filling material at the time of administration of the bone filling material is prevented. For the use of a kit for preparing PRP, the surface treatment agent and PRP are accommodated in the vessel for PRP, the bone filling material is impregnated in the accommodated surface treatment agent and PRP or the surface treatment agent and PRP accommodated in the vessel for PRP are coated onto the bone filling material, and as a result PRP is incorporated in the bone filling material. As one embodiment of the kit of the invention, the bone filling material which is coated or impregnated in advance with the surface treatment agent may be also used.

The vessel for preparing PRP, which is used for the impregnation step, may have a size to accommodate the surface treatment agent, PRP, and the bone filling material, and it can be appropriately prepared. Specifically, the volume of the vessel may be 1 to 50 mL, and it also may be 2 to 25 mL. Shape of the vessel is not specifically limited, and examples thereof include a column shape and a polygonal barrel shape of which one end is covered to prevent liquid spill. Further, a syringe shape vessel can be also used as a vessel to be used for the invention. Outlet of the syringe is covered by a seal or a cap (preferably a screw type). A well known syringe can be used as appropriate. When impregnation is performed by using such syringe type vessel, the bone filling material is added at the opening end of the syringe (i.e., plunger side) of which outlet is covered. After that, any one of the surface treatment agent and PRP or both of them (i.e., previously mixed) are injected. Order of adding the bone filling material and PRP to the syringe can be reversed. However, to obtain high functional effect of a growth factor included in PRP, it is preferable to add PRP after adding the bone filling material and the surface treatment agent. After that, the plunger is inserted from the opening end of the plunger side of the syringe, and pressure is applied to the extent that the seal or the cap is not taken off.
Accordingly, the impregnation step can be easily carried out under increased pressure, and the surface treatment agent and PRP can be quickly infiltrated. A well known material can be used as a material of the vessel for preparing PRP that is used for the infiltration step. It is preferably a material to which the surface treatment agent or a protein, etc. in PRP cannot easily adsorb. Such material is well known to a skilled person in the pertinent art, and examples include glass and polypropylene.

Examples of the vessel which is used for the coating step include a spray vessel. A well known spray vessel may be used. The coating step using the spray vessel may be carried out according to a well known method. For example, in a vessel having a flat plane to which the bone filling materials can be placed without overlap, the bone filling materials are aligned. Bottom of the vessel preferably has a grid shape to prevent fall of the bone filling materials. Then, by spraying above and below the grid, the bone filling materials can be coated with the surface treatment agent or PRP. The surface treatment agent and PRP may be added separately to the spray vessel, or they may be mixed and then added to the vessel. When coating is carried out by using the spray vessel, the liquid released from the vessel is a spray type. As such, viscosities of the surface treatment agent and PRP are preferably not so high. When they are too high, it is difficult to achieve homogeneous spray coating. A skilled person in the pertinent art may easily adjust the viscosity depending to the property of the surface treatment agent used, etc.

### EXAMPLE 1

In Example 1, the effect of the bone filling material impregnated with a surface treatment agent on cell proliferation was examined. Reagents used in Example 1 are as follows. As α-TCP, the product of TAIHEI CHEMICAL INDUSTRIAL CO., LTD. was used. As Otsuka distilled Water, the product of Otsuka Pharmaceutical Co., Ltd. was used. As L form serine, the product of NACALAI TESQUE, INC. was used. As dextran, the product of Meito Sangyo Co., Ltd. was used. As trehalose, the product of Hayashibara Biochemical Laboratories, Inc. was used. As succinic acid, the product of Kawasaki Kasei Chemicals Ltd. was used. As disodium succinate, the product of Wako Pure Chemical Industries, Ltd. was used. In order to produce the bone filling material, ZPrinter 406 (trade name) manufactured by ZCorporation was used.

### Step of producing tetrapod type bone filling material (Tetora Bone)

Tetrapod type bone filling material was produced by using ZPrinter 406 using α-TCP as a main raw material. The tetrapod type bone filling material was immersed in a sclerosing solution. For the sclerosing solution, 0.2 mol/L aqueous solution of succinic acid (pH 6) was used. After the immersion in the sclerosing solution, the tetrapod type bone filling material was washed twice with Otsuka distilled Water, and then dried for 12 hours under reduced pressure by using a low-temperature vacuum dryer (manufactured by Yamato Scientific Co., Ltd.).

### Step of infiltrating with surface treatment agent

Next, 1 g of the tetrapod type artificial bone obtained from the above step was immersed in 50 mL solution of the surface treatment agent. By keeping it at room temperature for 24 hours, infiltration with the surface treatment agent was performed. Further, it is also possible to coat the surface treatment agent according to a known method. After the infiltration with the surface treatment agent, preliminarily drying and sterilization treatments were carried out for 20 min under the environment of 121°C by using an autoclave. After that, the drying was carried out for 24 hours at room temperature and under reduced pressure. As a result, the Tetrapod type artificial bone (hereinbelow, also referred to as Tetra Bone or Tetrabone) was produced. Further, the drying may be performed under the environment of high temperature or low humidity condition. Further, as a control, a tetrapod type artificial bone which has not been immersed in the solution of the surface treatment agent was produced. The surface treatment agent (i.e., blocking agent) and its concentration used for the present step are described in Table 1.

### [Table 1]

**Table 1**

| Blocking agent | Concentration |
|---|---|
| Trehalose | 0.2 mol/L |
| L-serine | 0.2 mol/L |
| Dextran 40 | 5% by weight |

### Evaluation of biocompatibility of tetrapod type bone filling material (Tetora Bone)

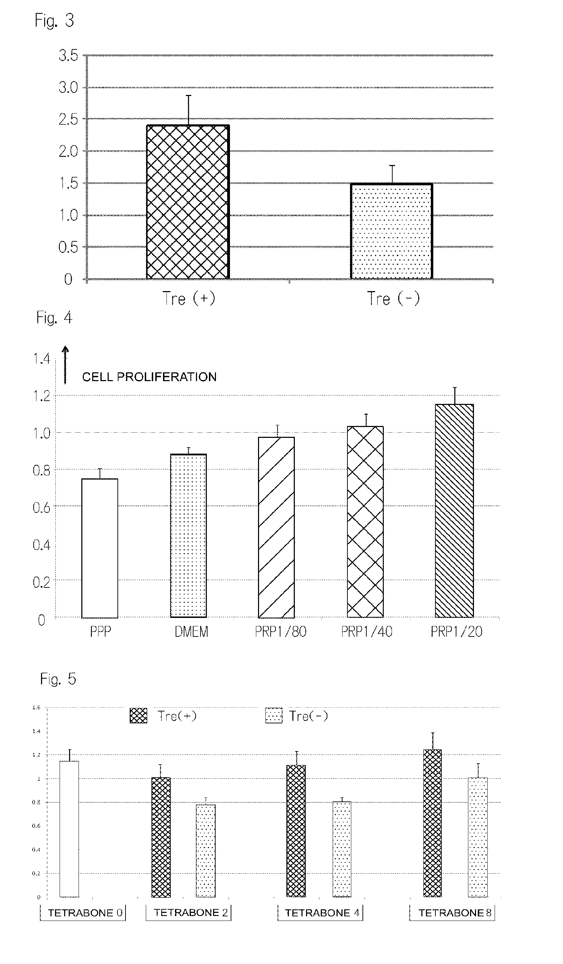

Co-culture of the tetrapod type bone filling material and cells was carried out, and biocompatibility evaluation was carried out. MC3T3-E1 cells, which are osteoblast-like cells, were used as the cell. As a culture medium, Dulbecco' s Modified Eagle Medium (D-MEM) containing 10% fetal bovine serum (FBS) and 1% penicillin and streptomycin was used. The MC3T3-E1 cells were cultured until the confluency, the culture medium was exchanged with a medium free of FBS (DMEM (FBS-)), and then the cells were seeded in a 96-multiwell plate to have 500 cells/well and incubated in an incubator (37°C, 5% CO₂) for 24 hours. After that, the tetrapod type artificial bone was added to each well to have 3 pieces/well and the culturing was further continuted for 24 hours. After the culturing, Cell counting Kit-8 (trade name) manufactured by DOJINDO LABORATORIES was added, and then a color reaction was carried out. After the reaction, the absorbance (450 nm) was measured using a microplate reader and the cell proliferation ability was determined. The results are shown in Fig. 5.

Fig. 2 is a graph, in place of a diagram, showing the effect of the tetrapod type artificial bone on cell proliferation, wherein the artificial bone had been immersed in each solution of the surface treatment agent including trehalose (Tre), L-serine (Ser), and dextran 40 (Dex) . The vertical axis of Fig. 2 indicates the absorbance at wavelength of 450 m. That is, higher value of the vertical axis means better cell proliferation. From the results of Fig. 2, it was found that the cells added with the tetrapod type bone filling material which had been immersed in each solution of the surface treatment agents including trehalose (Tre), L-serine (ser), and dextran 40 (Dex) showed better cell proliferation compared to the cells (C) not added with the bone filling material. On the contrary, it was found that the cells (non) added with the tetrapod type bone filling material which had not been immersed in the solution of the surface treatment agent showed poorer cell proliferation compared to the cells (C) not added with the bone filling material. Based on this result it was considered that, as the adsorption site for a growth factor in the artificial bone for the bone filling material is not blocked by the surface treatment agent, the growth factor contained in blood serum (FBS) adsorbs onto the artificial bone for the bone filling material so that the cell proliferation is inhibited. Therefore, as the adsorption site for a growth factor in the artificial bone for the bone filling material is blocked by using trehalose, L-serine, or dextran 40 as a solution of the surface treatment agent, it may be appropriately used as a bone filling material which does not inhibit the bone growing effect of a growth factor.

### EXAMPLE 2

### Determination of adsorptivity of growth factor

To determine that the bone filling material containing the surface treatment agent inhibits the adsorption of a growth factor, its effect on cell proliferation ability was examined by using the bone filling material treated with the surface treatment agent and the non-treated bone filling material. As a growth factor, PRP containing a growth factor was used.

### Preparation of PRP

Human blood sample was prepared and subjected to the first centrifuge (2100 rpm, 20°C, 10 min). After the centrifuge, the upper layer (platelet poor plasma: PPP) and the medium layer (buffy coat) were collected and stored in a single tube. After that, the second centrifuge (3400 rpm, 20°C, 10 min) was carried out. After the centrifuge, the upper layer (PPP) was collected.

Similar to Example 1, MC3T3-E1 cells, which are osteoblast-like cells, were used as the cell. As a culture medium, DMEM containing 1% FBS was used. The MC3T3-E1 cells were seeded in a 96-multiwell plate to have 500 cells/well and incubated in an incubator (37°C, 5% CO₂) for 24 hours. Thus-prepared PRP was diluted with DMEM containing 1% FBS to obtain 40 x (i.e., 1/40) dilution. 250 mg of the bone filling material (Tetrabone) which had been either treated (Tre (+)) or not treated (Tre (-)) with trehalose was added to 1.5 mL of diluted solution of PRP, and then allowed to stand for 1 hour. After that, the supernatant and the bone filling material (Tetrabone) were recovered. To the cells which had been cultured for 24 hours, the supernatant was added with 10 uL/well or the bone filling material (Tetrabone) was applied to each well, and then the cells were cultured for 48 hours. After the culturing, Cell counting Kit-8 (trade name) manufactured by DOJINDO LABORATORIES was added, and then a color reaction was carried out. After the reaction, the absorbance (450 nm) was measured using a microplate reader and the cell proliferation ability was determined. The results are shown in Fig. 3.

Fig. 3 is a graph, in place of a diagram, showing the effect of the trehalose treatment on adsorptivity of the bone filling material (Tetrabone) for a growth factor. The vertical axis of Fig. 3 indicates the absorbance at wavelength of 450 m. That is, higher value of the vertical axis means better proliferative activity of osteoblast cells, i.e., inhibited adsorption of the growth factor. From the results of Fig. 3, it was found that the cells added with the trehalose-treated bone filling material (Tetrabone) exhibits an increased cell proliferation activity compared to the cells added with the bone filling material (Tetrabone) that had not been treated with trehalose. Thus, it was recognized that the growth factor adsorptivity of the bone filling material (Tetrabone) is inhibited by the trehalose treatment.

### EXAMPLE 3

### Bone regeneration promoting effect of PRP

PRP's bone regeneration promoting effect was examined.

### Preparation of PRP

Human blood sample was prepared and subjected to the first centrifuge (2100 rpm, 20°C, 10min) . After the centrifuge, the upper layer (PPP) and the medium layer (buffy coat) were collected and stored in a single tube. After that, the second centrifuge (3400 rpm, 20°C, 10 min) was carried out. After the centrifuge, each of the upper layer (PPP) and the bottom layer (PRP) were collected.

Similar to Example 1, MC3T3-E1 cells, which are osteoblast-like cells, were used as the cell. The MC3T3-E1 cells were cultured until the confluency, the culture medium was exchanged with a medium free of FBS (DMEM (FBS-)), and then the cells were seeded in a 96-multiwell plate to have 500 cells/90 uL/well and incubated in an incubator (37°C, 5% CO₂) for 24 hours. After that, 10 uL of PRP (20 x dilution (PRP 1/20), 40 x dilution (PRP 1/40), or 80 x dilution (PRP 1/80)), PPP, or DMEM (FBS (-)) as a control was added to each well, and then cultured for 48 hours. After the culturing, Cell counting Kit-8 (trade name) manufactured by DOJINDO LABORATORIES was added, and then a color reaction was carried out. After the reaction, the absorbance (450 nm) was measured using a microplate reader and the cell proliferation ability was determined. The results are shown in Fig. 4.

Fig. 4 is a graph, in place of a diagram, showing the effect of PRP on the proliferation of MC3T3-E1 cells, which are osteoblast-like cells. The vertical axis of Fig. 4 indicates the absorbance at wavelength of 450 m. That is, higher value of the vertical axis means better proliferation of osteoblast cells, i.e., the effect of promoting bone regeneration. From the results of Fig. 4, it was found that the cell proliferation is better in the cells added with PRP in a concentration-dependent manner compared to the cells of a control (i.e., added with DMEM) . Thus, it was recognized that PRP increases the effect of promoting bone regeneration in a concentration-dependent manner.

### EXAMPLE 4

### Characteristics of co-culture of tetrapod type bone filling material (Tetora Bone) and PRP

In Example 4, the effect of the tetrapod type bone filling material impregnated with the surface treatment agent (trehalose) on the effect of promoting bone regeneration by PRP was determined.

Cell proliferation ability was examined by co-culture of the tetrapod type bone filling material (Tetrabone) impregnated with the trehalose solution (hereinbelow, referred to "Tre (+)") and PRP. As a control, the tetrapod artificial bone not impregnated with the trehalose solution ((hereinbelow, referred to "Tre (-)") was used. Similar to Example 1, MC3T3-E1 cells, which are osteoblast-like cells, were used as the cell. The MC3T3-E1 cells were cultured until the confluency, the culture medium was exchanged with a medium free of FBS (DMEM (FBS-)) , and then the cells were seeded in a 96-multiwell plate to have 500 cells/90 uL/well and incubated in an incubator (37°C, 5% CO₂) for 24 hours. After that, 10 uL of PRP (20 x dilution (PRP 1/20)) and pre-determined number (0, 2, 4 or 8 pieces) of the tetrapod type bone filling material were added to each well, and cultured for 48 hours. After the culturing, Cell counting Kit-8 (trade name) manufactured by DOJINDO LABORATORIES was added, and then a color reaction was carried out. After the reaction, the absorbance (450 nm) was measured using a microplate reader and the cell proliferation ability was determined. The results are shown in Fig. 5.

Fig. 5 is a graph, in place of a diagram, showing the effect of the tetrapod type bone filling material (Tetrabone) on cell proliferation ability of PRP, wherein the bone filling material was impregnated with an aqueous solution of trehalose. The vertical axis of Fig. 5 indicates the absorbance at wavelength of 450 m. That is, higher value of the vertical axis means better proliferation, i.e., the effect of promoting bone regeneration. When the Tre (-) artificial bone and the Tre (+) artificial bone are compared to each other from the results of Fig. 5, it was found that the Tre (+) artificial bone has better cell proliferation. As such, it was recognized that the PRP's bone regeneration effect can be effectively obtained by using the Tre (+) bone filling material. Further, from Fig. 5, it was found that more tetrapod type bone filling materials show better cell proliferation. Therefore, it is believed that the bone filling material infiltrated with the surface treatment agent can be used as a good basis for cell proliferation, and by using it in combination with PRP, the bone regeneration effect can be effectively promoted.

From the results above, it was found that the cell proliferation is promoted by impregnating the bone filling material with the surface treatment agent. Specifically, the bone filling material infiltrated with the surface treatment agent can effectively promote the bone regeneration. It was furthermore shown that, by impregnating the bone filling material with the surface treatment agent, not only the PRP's bone regeneration effect can be obtained but also the bone regeneration effect can be promoted.

### EXAMPLE 5

### Determination of effect of surface treatment of the bone filling material on bone regeneration in living body

In order to determine the effect of the surface treatment of the bone filling material on bone regeneration in a living body, a rat model having femur bone-defect was prepared. The tetrapod type bone filling material of which surface has not been treated or the tetrapod type bone filling material with the trehalose surface treatment was implanted in the bone defect part, and then the degree of bone regeneration was evaluated according to histological analysis. To the defect part (diameter of 2 mm and depth or 2.5 mm) produced on the femur bone of a rat (female, 10.5 to 12.5 week-old), 11 to 12 pieces of the tetrapod type bone filling material were implanted. One week later, the animal was scarified and the femur bone including the defect part was harvested. The sample was fixed with formalin and decalcified using Plank-Rychlo' s solution to give a tissue section, which was then stained with hematoxylin eosin. Further, to evaluate the degree of bone regeneration, a grid having one hundred blocks (diameter of one memory is 25 µm) was formed on part of an image of the tissue which is surrounded by the bone filling material and the bone is ultimately produced, and then the number of blocks which are confirmed to have progressive bone regeneration was counted (Fig. 6B and Fig. 6D). The results are shown in Fig. 6.

Fig. 6 includes photographs, in place of a drawing, showing the effect of the surface treatment of the bond filling material on bone regeneration in a living body. Fig. 6A is a photograph, in place of a drawing, showing the tissue section of the rat femur bone to which the tetrapod type bone filling material without surface treatment has been implanted. Fig. 6B is a photographic image, in place of a diagram, wherein a grid consisting of one hundred blocks is formed on the enlarged image of the tissue section shown in Fig. 6A. Fig. 6C is a photographic image, in place of a diagram, showing the tissue section of a rat femur bone in which the bone filling material with the surface treatment has been implanted. Fig. 6D is a photographic image, in place of a diagram, wherein a grid consisting of one hundred blocks is formed on the enlarged image of the tissue section shown in Fig. 6C.

As a result, it was confirmed that significant bone regeneration is obtained from the tetrapod type bone filling material which had received the surface treatment (Fig. 6C and Fig. 6D) (the portion indicated by the arrow in Fig. 6D corresponds to the portion from which bone regeneration was found). However, no bone regeneration was observed from the tetrapod type bone filling material with no treatment (Fig. 6A and Fig. 6B).

Furthermore, the same analysis was carried out with increased trial number, and statistical treatment was performed with regard to the bone regeneration. The trial number was 24 times for the animal implanted with the tetrapod type bone filling material with no treatment, and 18 times for the tetrapod type bone filling material with the surface treatment. Results are shown in Fig. 7. The vertical axis of Fig. 7 represents the amount of regenerated bone (i.e., amount of the newly generated artificial bone). From the results of Fig. 7, it was found that the surface treatment of the bone filling material specifically promotes the bone regeneration (Fig. 7) . Based on this result, it is believed that excess adsorption of a factor required for bone regeneration on the surface of the bone filling material is prevented by the surface treatment.

Based on the above, it was shown that the bone regeneration is promoted by using the bone filling material impregnated with the surface treatment agent. In other words, the bone filling material infiltrated with the surface treatment agent shows inhibited adsorption of a factor that is required for bone regeneration on the bone filling material. Therefore, it was found that the bone filling material infiltrated with the surface treatment agent can effectively promote bone regeneration.

### INDUSTRIAL APPLICABILITY

The bone filling material of the invention can be used by injection to bone deformation sites, bone defect sites, or osteoporosis sites, or filling in bone defect sites, etc. Thus, the bone filling material of the invention may be used in the field of medical and pharmaceutical industry, etc.

### EXPLANATION OF REFERENCE NUMERALS

- 11: Bone filling material
- 12: Protruding part
- 13: Tip part

## Claims

1. A bone filling material comprising:
an artificial bone for the bone filling material containing a calcium-based material, a surface treatment agent coated on the artificial bone for the bone filling material, or a surface treatment agent impregnated in the artificial bone for the bone filling material,
wherein adsorptivity of a growth factor is controlled by the surface treatment agent, and as a result, inhibition of the activity of the growth factor that is caused by adsorption of the growth factor on the artificial bone for the bone filling material at the time of administration of the bone filling material is prevented.

2. The bone filling material according to Claim 1,
wherein the growth factor is a bone growth factor contained in body fluid which infiltrates an administration site to which the bone filling material is administered.

3. The bone filling material according to Claim 1,
wherein the growth factor is included in platelet rich plasma.

4. The bone filling material according to Claim 1,
wherein the artificial bone for the bone filing material containing a calcium-based material is a sintered body.

5. The bone filling material according to Claim 1,
wherein the weight ratio between the artificial bone for the bone filling material and the surface treatment agent is 1×10² : 1 to 1×10¹⁰ : 1.

6. The bone filling material according to Claim 1,
wherein the calcium-based material is one kind or a mixture of more than one kind selected from hydroxyl apatite, carbonic acid apatite, β-TCP, α-TCP, calcium metaphosphate, tetra-calcium phosphate, calcium hydrogen phosphate, calcium dihydrogen phosphate, calcium pyrophosphate, calcium carbonate, calcium sulfate, the salts thereof, or the solvates thereof.

7. The bone filling material according to Claim 1, in which the artificial bone for the bone filling material is any one of a granule type artificial bone, a block type artificial bone, an order made artificial bone, an implant for dental root, or a sclerosing type artificial bone.

8. The bone filling material according to Claim 1,
wherein the artificial bone for the bone filling material is a granule type, has a plurality of protruding parts, and the diameter size of the sphere which can accommodate the artificial bone for the bone filling material is 1×10⁻² mm to 5 mm.

9. The bone filling material according to Claim 1,
wherein the surface treatment agent is any one kind or a mixture of more than one kind of a chelating agent, a capping agent, a coupling agent, and a cross-linking agent.

10. The bone filling material according to Claim 1,
wherein the surface treatment agent is a chelating agent, which is one kind or a mixture of more than one kind selected from a group consisting of a linear-chain polyphosphoric acid chelating agent, gluconic acid, asparaginic acid, ethylenediamine tetraacetic acid, metaphosphoric acid, citric acid, malic acid, nitrilo triacetic acid, and methyl glycine diacetic acid.

11. The bone filling material according to Claim 1,
wherein the surface treatment agent is a capping agent, which includes capping of any one or more of an amino acid, a peptide, a polysaccharide, a disaccharide, lectin, proteoglycan, a glycoprotein, and a glycolipid.

12. The bone filling material according to Claim 1,
wherein the surface treatment agent is a coupling agent, which is an aluminate-based coupling agent, a titanol-based coupling agent, or a silanol-based coupling agent.

13. The bone filling material according to Claim 1,
wherein the surface treatment agent is trehalose, serine, or dextran.

14. The bone filling material according to Claim 1,
wherein the surface treatment agent is trehalose.

15. The bone filling material according to Claim 1,
wherein the bone filling material further comprises a bone growth agent coated on the artificial bone for the bone filling material or a bone growth agent impregnated in the artificial bone for the bone filling material.

16. The bone filling material according to Claim 1,
wherein the bone filling material further comprises platelet rich plasma coated on the artificial bone for the bone filling material or platelet rich plasma impregnated in the artificial bone for the bone filling material.

17. A method of producing a bone filling material comprising:
infiltrating a surface treatment agent into an artificial bone for a bone filling material; and
incorporating a bone growth agent in the artificial bone for the bone filling material to which a surface treatment agent has been infiltrated,
wherein the infiltration of a surface treatment agent into an artificial bone for a bone filling material is to coat the artificial bone for the bone filling material with a surface treatment agent or to impregnate the artificial bone for the bone filling material in a solution of a surface treatment agent,
the incorporation of a bone growth agent is to coat the artificial bone for the bone filling material to which a surface treatment agent has been coated or impregnated with a bone growth agent or to impregnate the artificial bone for the bone filling material to which a surface treatment agent has been coated or impregnated in a solution of a bone growth agent, and
the artificial bone for the bone filling material contains a calcium-based material.

18. A medical kit comprising:
a bone filling material, a surface treatment agent, platelet rich plasma, and a vessel for preparing platelet rich plasma,
wherein, for using the medical kit, the surface treatment agent and platelet rich plasma are accommodated in the vessel for platelet rich plasma,
the bone filling material is impregnated with the accommodated surface treatment agent and platelet rich plasma or the bone filling material is coated with the surface treatment agent and platelet rich plasma accommodated in the vessel for platelet rich plasma so that the platelet rich plasma is incorporated in the bone filling material, and
the adsorptivity of a growth factor in platelet rich plasma is controlled by the surface treatment agent, and as a result, inhibition of the activity of the growth factor that is caused by adsorption of the growth factor on the artificial bone for the bone filling material at the time of administration of the bone filling material is prevented.
